(19) Europäisches Patentamt
European Patent Office
Office européen des brevets

(11) Publication number: **0 339 217 B1**

# (12) EUROPEAN PATENT SPECIFICATION

(45) Date of publication of patent specification: **21.09.94**

(51) Int. Cl.⁵: **C07K 1/12**, G01N 33/68, C07F 9/50, C07F 9/547

(21) Application number: **89103906.7**

(22) Date of filing: **06.03.89**

## (54) Specific peptide bond cleavage by organic tertiary phosphines with nucleophilic side chains.

(30) Priority: **01.04.88 US 176571**
**01.04.88 US 176573**

(43) Date of publication of application:
**02.11.89 Bulletin 89/44**

(45) Publication of the grant of the patent:
**21.09.94 Bulletin 94/38**

(84) Designated Contracting States:
**AT BE CH DE ES FR GB GR IT LI NL SE**

(56) References cited:
**US-A- 3 628 910**
**US-A- 3 754 035**

**JOURNAL OF THE AMERICAN CHEMICAL SOCIETY, vol. 102, no. 4, 13th February 1980, pages 1436-1439, American Chemical Society; E.J. COREY et al.: "Stereospecific total synthesis of a "Slow Reacting Substance" of anaphylaxis, leukotriene C-1"**

(73) Proprietor: **CARTER-WALLACE, INC.**
**1345 Avenue of the Americas**
**New York, N.Y. 10105 (US)**

(72) Inventor: **Hinman, Lois M.**
**126 Harwood Avenue**
**North Tarrytown New York 10591 (US)**
Inventor: **Miller, Libby S.**
**250 W. 94th Street**
**New York New York 10025 (US)**

(74) Representative: **Wächtershäuser, Günter, Prof. Dr.**
**Patentanwalt**
**Tal 29**
**D-80331 München (DE)**

**EP 0 339 217 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Field of the Invention

The invention relates to novel nucleophilic tertiary organophosphines and the specific cleavage of peptide bonds by nucleophilic tertiary organophosphines. This invention is of particular utility in providing chemical agents for use in the cleavage of proteins and the determination of the amino acid sequence of proteins.

### Description of the Prior Art

Determination of the sequence of amino acids in a protein requires a reproducible method of cleaving polypeptide chains into fragments. A strategy for amino acid sequence analysis requires that a protein be cleaved into fragments by at least two different specific methods so that sequences of overlapping peptide fragments can be obtained.

Various methods for cleaving the polypeptide chains are known, e.g. enzymatic and chemical, which are discussed as follows. The ability of proteolytic enzymes such as trypsin, chymotrypsin, thermolysin and pepsin to cleave proteins at unique sites based upon amino acid sequence is well known to those skilled in the art. Of these enzymes, trypsin is the most specific protease cleaving a polypeptide on the C-terminal side of lysine or arginine residues. Chymotrypsin and pepsin cleave a polypeptide at the C-terminal side of phenylalanine, tryptophan or tyrosine residues while thermolysin cleaves at the N-terminal side of leucine, isoleucine or valine residues. [Lehninger, A.L., Biochemistry 2nd edition, 1975, Worth Publishers].

Of the chemical methods for cleaving polypeptide chains, the most common involves the use of cyanogen bromide which cleaves on the C-terminal side of methionine residues. [Gross, E., The cyanogen bromide reaction, Methods in Enzymology, 11, 238, 1967]. Other chemical cleavage reactions include cleavage at tryptophanyl residues by N-bromosuccinimide [Schecter, Y., Patchornik, A. and Burstein, Y, Selective chemical cleavage of tryptophanyl peptide bonds by oxidative chlorination with N-bromosuccinimide, Bio-chemistry, 11 5071, 1976]. This reaction tends to be relatively inefficient in proteins, cleaving only 10 to 50% of the tryptophanyl peptide bonds in proteins. Additional chemical cleavage agents which react primarily at tryptophanyl residues have been described. These include 2-(2-nitrophenyl-sulfonyl)-3-methyl-3-bromo -indolenine [Fontana, A., Modification of Tryptohan with BNPS-skatole, Meth. Enzymology, 25, 29, 1972], 2,4,6-tribromo-4-methyl-cyclohexadione [Burstein Y. and Patchornik, A., Selective chemical cleavage of tryptophanyl peptide bonds in peptides and proteins, Biochemistry, 11, 4641, 1972], o-iodobenzoic acid [Mahoney, W.C. and Hermodson, M.A., High yield of cleavage of tryptophanyl peptide bonds by o-iodobenzoic acid, Biochemistry, 18, 3810, 1979] and N-chlorosuccinimide [Schecter, Y., Patchornik, A. and Burstein, Y., Selective chemical cleavage of tryptophanyl peptide bonds by oxidative chlorination with N-chlorosuccinimide, Biochemistry, 15, 5071, 1976]. As with the N-bromosuccinimide reaction, all of these agents result in relatively inefficient cleavage and further result in the chemical modification of some other amino acid residues. Furthermore, hydroxylamine is known to cleave polypeptides at asparaginyl-glycyl peptide bonds [Bornstein, P. and Balian, G., Cleavage of Asn-Gly bonds with hydroxylamine, Meth. Enzymology, 47, 132, 1977]. All of these cleavage methods work optimally only on reduced and alkylated proteins rather than on proteins with intact disulfide bonds.

It is also known that cleavage of polypeptides at cysteine or serine can be accomplished by conversion of the amino acid residue to dehydroalanine and subsequent hydrolysis of the dehydroalanine-containing polypeptide in acid. [Witkop, B. and Ramachandran, L. K. Progress in nonenzymatic selective modification and cleavage of proteins, Metabolism, 13, 1016, 1064; Patchornik, A. and Sokolooslky, M., Nonenzymatic cleavages of peptide chains at cystine and serine residues through their conversion into dehydroalanine residues, J. Amer. Chem. Soc., 86, 1206, 1964; Sokoloosky, M. Sadeh, T. and Patchornik, A., Nonenzymatic cleavage of peptide chains at the cystine and serine residues through their conversion to dehydroalanine (DHAL). II. The specific chemical cleavage of cysteinyl peptides, J. Amer. Chem. Soc., 86, 1212, 1964] The only chemical agent known prior to the invention herein described which would uniquely cleave a polypeptide at cystine residues is cyanide. [Cartsimpoolas, N. and Wood, J. L. The reaction of cyanide with bovine serum albumin, J. Biol. Chem., 239, 4132, 1964; Cartsimpoolas, N. and Wood, J.L., Specific cleavage of cystine peptides by cyanide, J. Biol. Chem., 241, 1790, 1966] Cyanide reacts with cystine to yield a sulfhydryl group and a thiocyano group. The thiocyano-containing derivative will cyclize at a pH of 8 or below and then undergo hydrolysis to cleave the peptide bond. Cleavage at cysteine or cystine residues can be accomplished by reacting a polypeptide with 2-nitro-5-thiocyano-benzoic acid. [Jacobson, G. R., Schaffer, M. H. Stank, G. R., Vanaman, T. C., Specific chemical cleavage in high yield at the amino peptide

bonds of cysteine and cystine residues. J. Biol. Chem., 248, 6583, 1973]. The N-terminus of the polypeptide is blocked by this method and requires additional treatment with a nickel and sodium borohydride catalyst to convert the modified amino acid residue to alanine. Cleavage of phenylalanyl -seryl and phenylalanylthreonyl peptide bonds by cyanide and cyano-derivatives has also been reported (Witkop, B and Ramachandran, L.K., Progress in Nonenzymatic Selective Modification and Cleavage of Proteins, Metabolism, 13 1016,1064). However, because of a lack of specificity in the cleavage reaction and concommitant modifications of other amino acid residues by cyanide, this reaction is only rarely used as a cleavage method in protein chemistry.

Certain tertiary organophosphines have been described as having the ability to alter the configuration of keratin fibers and, under certain conditions, cause depilation of hair. U.S. Patent No. 3,489,811 to Drucker et al. discloses certain tertiary organic phosphines which are suitable for cosmetic uses such as in hair-waving compositions. In addition, that patent discloses and claims processes for producing a substantially odor-free tertiary phosphine. The use of particular organic phosphines to deform keratin fibers or cause depilation is disclosed in U.S. Patent No. 3,628,910 to Grayson. The Grayson patent teaches that the deformation and depilation effects on keratin fibers by particular organic phosphines are the result of the rupture and reformation of cystine disulfide bond linkages which exist between polypeptides of keratin fibers. Furthermore, the ability of certain unsymmetrical tertiary organophosphines to effect depilation is disclosed in U.S. Patent No. 3,754,035 to Grayson. Here, too, it is taught that the effect of such organophosphines is to cleave disulfide bonds of the hair keratin. The ability for organic phosphines to reduce disulfide bonds by acting as reducing agents has been shown using model compounds. In addition, the use of tributylphosphine as a reducing agent in protein research has been reported. [Ruegg, U.T. and Rudingo, J. Reductive Cleavage of Cystine Disulfides with Tributylphosphine, Methods in Enzymology, 47, 111-126] However, as far as we are aware, until the present invention, it was not know that nucleophilic tertiary organophosphines would effect specific peptide bond cleavage.

Therefore, it is a primary object of this invention to provide nucleophilic tertiary organophosphines which are capable of effecting peptide bond cleavage. It is a further specific object of this invention to provide nucleophilic tertiary organophosphines which are capable of effecting peptide bond cleavage in a specific manner. Still further, it is an object of this invention to provide methods to be used with such nucleophilic tertiary organophosphines to effect such peptide bond cleavage. Additional objects and advantages of the present invention will be set forth in part in the description which follows and in part will be obvious from the description or may be learned by practice of the invention as hereinafter described and claimed.

## Summary of the Invention

The novel nucleophilic tertiary organophosphines of the present invention can be represented by the formula:

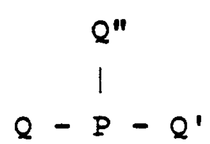

Wherein Q is

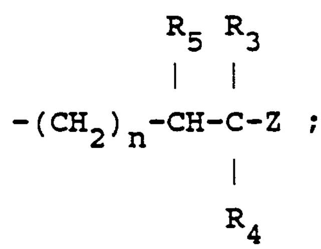

Q' is Q or Q''
Q'' is

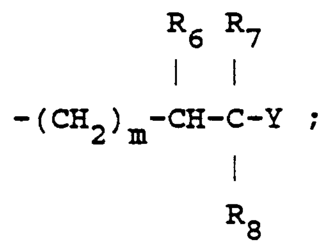

Y and Z are, $-NR_{10}R_{11}$ or $-OR_{12}$ ;
$R_{10}$ and $R_{11}$ taken with the N form imidazolyl, pyrridonyl or pyridyl rings;
$R_{12}$ is

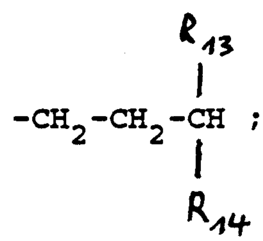

$R_{13}$ is H or -OH;
$R_{14}$ is $-(CH_2)_t-CH_2OH$;
n,m and t are independently 0-3;
$R_3$-$R_8$ are independently H or alkyl ($C_1$-$C_4$);
with the provisos that: Z and Y are not the same for any given compound.

In addition to the foregoing novel nucleophilic tertiary organophosphines present invention is also directed to the novel use of nucleophilic tertiary organophosphines as specific peptide bond cleavage agents. The nucleophilic tertiary organophosphines used in this invention to specifically cleave peptide bonds at cysteine as hereinafter described can be represented by the formula:

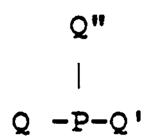

Wherein Q is

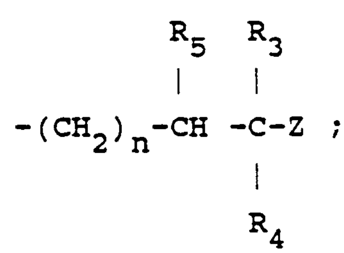

Q' is Q or Q''
Q'' is

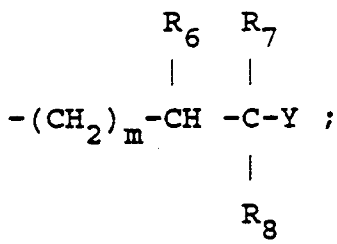

Y and Z are -OH, $-NR_{10}R_{11}$ or $-OR_{12}$ ;
$R_{10}$ and $R_{11}$ are independently H or alkyl ($C_1$-$C_4$);or
$R_{10}$ and $R_{11}$ taken with N form pyrridonyl, pyridyl, piperidinyl, imidazolyl or pyrrolidinyl; or
$R_{11}$ is acetyl, carbamyl or guanyl when $R_{10}$ is H;
$R_{12}$ is

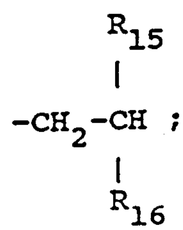

$R_{15}$ is H or -OH ;
$R_{16}$ is $-(CH_2)_t-CH_2OH$ ;
n, m and t are independently 0-3.

## DETAILED DESCRIPTION OF THE INVENTION

### PREPARATION OF NUCLEOPHILIC TERTIARY ORGANOPHOSPHINES

The nucleophilic tertiary organophosphines of the present invention are prepared by procedures well known to those skilled in art. See, for example, J. Org Chem. 26, 5138 (1961); U.S. Patent 2,803,597 to Stiles, et al; and U.S. Patent 3,754,036 to Grayson. A. The following Examples will serve to illustrate the invention in more detail and should not be construed to limit the scope of the invention in any way. The general procedure is to react phosphine ($PH_3$) in a pressurized vessel with a terminally ethylenic unsaturated amine or alcohol in the presence of a free radical initiator such as 2,2' azobis(2-methylbutyronitrile) (hereinafter sometimes referred to as ABN). After separation of the desired product (primary or secondary phosphine) from the reaction mixture by distillation, it is dissolved in a suitable solvent and reacted in an inert atmosphere with a terminally ethylenically unsaturated alcohol or amine, again in the presence of a free radical initiator. Typical of the terminally ethylenic unsaturated amines are 3-allyl-1-p-morpholine, 3-allyl-1-imidazole, 3-allyl-1-pyrollidone and 3-N,N-diethylaminopropene. Typical of the terminally ethylenic alcohols are 3-hydroxypropene, ally-2-hydroxyethyl ether and 6,7-dihydroxy-4-oxahept-1-ene (3-allyloxy-1,2-propane-diol).
Whether Q is the same as Q″ in the formula:

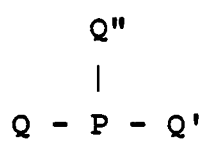

is dependent on the reaction ratios used in the above described reactions. Product purity of the compounds produced according to the above process can be established by microanalysis and $^{31}P$ NMR spectroscopy.

Example 1

A. 3-N,N-Diethylaminopropylphosphine

A one gallon autoclave containing 500ml. 2-propanol was pressurized to 600 psi with $PH_3$ and heated to 85°C. A mixture of 500g (4.4 moles) N,N-diethylallylamine, 500g 2-propanol and 10g (.05 moles) 2,2′-azobis(2-methylbutyronitrile) was fed into the system to a total charge of 1500g. The reaction was maintained at 600 psi for 2 hours and then heated at 85° for 1 hour. The autoclave was vented and the primary phoshine (229g, 1.54 moles) was separated from the solvent and secondary & tertiary phosphine products by distillation.

B. 3-Diethylaminopropyl bis (3-imidazolyl propyl)phosphine

18.5g of 3-N,N-diethylaminopropylphosphine from part A above was dissolved in 150 ml of 2-propanol in a reaction flask containing an inert atmosphere. One-half gram 2,2′-azobis(2-methylbutyronitrile) was added to the solution which was then heated in an oil bath to 55-60°C. A solution of 28.1g of allylimidazole in 30ml of 2-propanol was added dropwise to the reaction mixture over a period of 45 minutes keeping the temperature of the reaction mixture between 55-60°C. The reaction mixture was then heated to 75°C for 6 hours. Then, the solvent, starting materials and intermediates were stripped off under high vacuum. The product (27g),3-diethylaminopropylbis(3-imidazolylpropyl)phosphine, remained in the distillation pot at 200°C and 0.1mm Hg. Further purification of the product, if desired, could be accomplished by distillation using a bulb-to-bulb apparatus (e.g., Kugelrohr) at 200°C and 0.1mm Hg.

Example 2

3-Diethylaminopropylbis(2-hydroxyethyloxy-3-propyl)phosphine 14.2g of 3-N,N-diethylaminopropylphosphine (prepared as described in Example 1, Part A above) was dissolved in 150 ml of 2-propanol in a reaction flask containing an inert atmosphere. One-half gram 2,2′-azobis(2-methylbutyronitrile) was added to the solution and the solution was heated to 70°C. A solution containing 22.4g of allylhydroxyethylether in 50ml of 2-propanol was added dropwise to the reaction mixture over a 45 minute period while maintaining the temperature of the reaction mixture between 70-80°C. The temperature of the reaction mixture was then maintained at 70-80°C for an additional 6 hours with the addition of 0.5g of ABN at 3.5 hours. The product, 21g of 3-diethylaminopropyl-bis(2-hydroxy-ethoxy-3-propyl)phosphine, was separated from the solvent, starting materials and intermediates and purified as described in Example 1.

Examples 3,4 and 5

The tertiary phosphines listed in Table 1 were prepared from the respective aminoalkyl or cycloaminoalkyl phosphine intermediates and alkenols using the process described in Example 1.

Example 6

A. 3-Hydroxypropylphosphine

3-Hydroxypropy(phospine was prepared from $PH_3$ and allyl alcohol in the presence of 2,2′-azobis(2-methy-lsobutyronitrile) essentially as in Example 1A.

B. 3-Hydroxypropyl bis(3-imidazolylpropyl) phospine (6) (mixture with #5, above).

12.0g of the reaction product of Part A, above, was dissolved in 150ml of 2-propanol in a reaction flask under an inert atmosphere. Then, 0.5g of ABN was added and the mixture was heated to 50-55°C. A mixture containing 28.1g of 3-allylimidazole in 30ml of 2-propanol was added dropwise to the reaction mixture over a 30 minute period. The reaction mixture was then heated at 80°C for a total of 6 hours with the addition of 0.2g ABN at 3 hours. The reaction produced an inseparable mixture of 20% 3-imidazolylpropylbis (3-hydroxy-propyl)phosphine (5) and 80% 3-hydroxypropylbis(3-imidazolylpropyl)phosphine (6) which were separated from the starting material, intermediates and solvent essentially as described in Example 1.

TABLE 1

| Example | Phosphine Intermediate | Alkenol | Tertiary Phosphine Product |
|---|---|---|---|
| 3 | $(C_2H_5)_2N-(CH_2)_3-PH_2$ | $H_2C=CH-CH_2-O-CH_2-CH(OH)-CH_2OH$ | $(C_2H_5)_2N-(CH_2)_3-P-[(CH_2)_3-O-CH_2-CH(OH)-CH_2OH]_2$ |
| 4 | 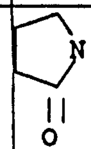 $-(CH_2)_2-PH_2$ | $H_2C=CH-CH_2-O-CH_2-CH(OH)-CH_2OH$ | 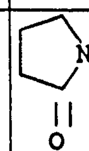 $-(CH_2)_2-P-[(CH_2)_3-O-CH_2-CH(OH)-CH_2OH]_2$ |
| 5 | 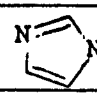 $-(CH_2)_3-PH_2$ | $H_2C=CH_2-CH_2-OH$ | 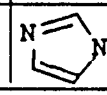 $-(CH_2)_3-P-(CH_2-CH_2-CH_2-OH)_2$ |

TABLE 2

| Example | Tertiary Phosphine Product | Theory C | Theory H | Theory N | Theory P | Found C | Found H | Found N | Found P |
|---|---|---|---|---|---|---|---|---|---|
| 1 | 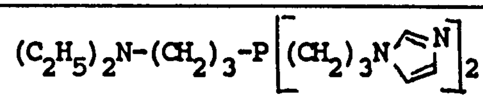 | 59.82 | 9.52 | 18.35 | 8.12 | 60.85 | 9.60 | 18.71 | 8.00 |
| 2 | $(C_2H_5)_2N-(CH_2)_3-P-[(CH_2)_3-O-CH_2CH_2OH]_2$ | 58.07 | 10.90 | 3.99 | 8.57 | 57.80 | 10.22 | 3.88 | 8.63 |
| 3 | $(C_2H_5)_2N-(CH_2)_3-P[(CH_2)_3-O-CH_2-CH(OH)-CH_2OH]_2$ | 55.45 | 10.29 | 3.40 | 7.50 | 55.39 | 10.14 | 3.33 | 7.15 |
| 4 | 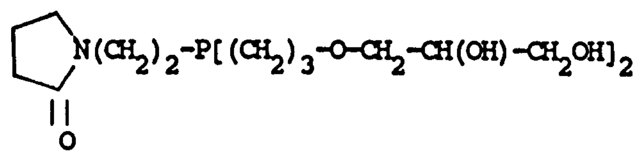 | 52.81 | 8.86 | 3.42 | 7.56 | 52.64 | 9.01 | 2.78 | 6.08 |
| 5 | 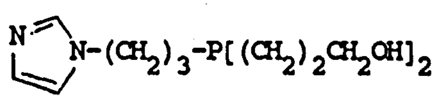 | 55.81 | 8.96 | 10.25 | 11.99 | 55.82 | 8.67 | 12.15 | 10.75 |
| 6 | 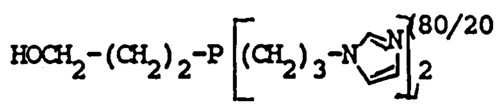 (80/20 mixture w/5) | 58.43 | 8.17 | 18.17 | 10.04 | 58.16 | 7.77 | 18.05 | 9.70 |

The reaction products of Example 1-6 were verified by elemental analysis and $^{31}$PNMR and the results are listed in Table 2.

Cleavage of Peptide Bonds by Nucleophilic Tertiary Organo Phosphines

Cleavage of peptide bonds in proteins by tertiary organophosphines was studied by treatment of proteins at concentrations ranging from about 0.1 to 5 mg/ml and in a pH range of about 3.0 to 11.0. The results of digestion of the proteins were determined by sodium dodecyl sulfate polyacrylamide gel electrophoresis (SDS-PAGE) according to methods well known to those skilled in that art.

For example, such methds are described by Weber, K. and Osborne, M., The Proteins, H. Neurath and R.L. Hill, editors, Academic Press, New York 19 75, Vol. I 3rd Ed., pp 179-223. The protein bands were identified by staining using either Coomassie Blue-R or Gel-Code silver stain. Specific examples of such cleavage are shown in the following examples which will serve to illustrate the invention in more detail and should not be construed to limit the scope of the invention in any way.

Example 7

Cleavage of hair keratin by N,N-diethylamiopropyl-bis-hydroxypropyl)Phosphine

The breakdown of hair keratin by N,N-diethylaminopropyl-bis(3-hydroxypropyl)phosphine as compared with thioglycolate was studied. 200mg of chopped human hair was digested at pH 10.5, 37° C for 4 hours in 10 ml of 8M urea containing 0.2M N,N-diethylaminopropylbis(3-hydroxypropyl)phosphine or 0.2M sodium thioglycolate. Following digestion, the hair proteins were acetylated by reaction with iodoacetic acid according to the procedure described by Shecter, et al., [J. Invest. Derm. 52 ,57 (1969)]. Samples of the digestion products were then analyzed by SDS-PAGE according to the procedure discussed above. Figure 1 is a representation of such a gel comparing the digestion of hair by N, N-diethylaminopropylbis(3-hydroxypropyl)phosphine and by sodium thioglycolate. As is shown in Figure 1, digestion of hair by N,N-diethylaminopropylbis(3-hydroxypropyl)phosphine produces lower molecular weight fragments than those produced by reduction of disulfide bonds alone (i.e., digestion by thioglycolate).

Example 8

Cleavage of Bovine Serum Albumin (BSA) by N,N-diethylaminopropylbis(3-hydroxypropyl)phosphine

The ability of N, N-diethylaminopropylbis(3-hydroxypropyl)phosphine to cleave BSA was studied and compared with effects of the disulfide bond reducing agents thioglycolate (TG) and 2-mercaptoethanol-(EtSH)
on BSA. Solutions of BSA at 10mg/ml in .1M tris buffer at pH 8.0 were combined with equal volumes of 0.2M N,N-diethylaminopropylbis(3-hydroxypropyl)-phosphine, sodium thioglycolate or 2-mercaptoethanol, boiled for 15 minutes and then mixed 1:1 with SDS sample buffer without reducing agent.

Figure 2 is a representation of a SDS-PAGE gel showing BSA treated with thioglycolate and BSA treated with N,N-diethylaminopropylbis(3-hydroxypropyl)phosphine. Treatment of BSA with EtSH or TG results in the production of a 66K monomer. Treatment of BSA with N,N-diethylaminopropyl-bis(3-hydroxypropyl) -phosphine results in cleavage of the 66K monomer into smaller peptide fragments indicating disruption of covalent bonds. There is no cleavage of covalent bonds in the 66K monomer by either EtSH or TG.

Example 9

Cleavage of Disulfide Bond Reduced Bovine Serum Albumin (BSA) by N,N-diethylaminopropyl-bis (3-hydroxypropyl)phosphine.

The effect of prior reduction of disulfide bonds in BSA upon subsequent cleavage by N,N-diethylaminopropylbis(3-hydroxypropyl)phosphine was studied. A 10 mg/ml sample of BSA was mixed with an equal volume of 0.2M 2-mercaptoethanol or thioglycolate and heated in boiling water for 15 minutes. The reaction mixtures were made 0.2M in N,N-diethylaminopropylbis(3-hydroxypropyl)phosphine and the reaction mixture treated as Example 8.

The results of this study are shown in Figure 2 which is a representation in a SDS-PAGE gel. As shown in Figure 2, no peptide bond cleavage by N,N-diethylaminopropylbis(3-hydroxypropyl)phosphine is observed when the BSA is pretreated with thioglycolate. These results indicate that intact disulfide bonds are required to effect cleavage of proteins by the compounds of this invention.

Example 10

Treatment of Sperm Whale Myoglobin with N, N-diethylaminopropylbis(3-hydroxypropyl)phosphine

The ability of N,N-diethylaminopropylbis (3-hydroxypropyl)phosphine to cleave sperm whale myoglobin was studied. Sperm whale myoglobin does not contain any cysteine residues. Specifically, the protein was treated at a concentration 10 mg/ml with 0.2m mercaptoethanol or phosphine as in Example 8.

SDS-PAGE showed that sperm whale myoglobin was not cleaved by N,N-diethylaminopropylbis-(3-hydroxy -propyl)phosphine.

Example 11

Cleavage of other Cystine Containing Proteins

Using the same procedure as in Example 8, the following proteins, all containing cystine residues, were treated with N,N-diethylaminopropylbis(3-hydroxy- propyl)phosphine solutions and the products analyzed by SDS-PAGE: alcohol dehydrogenase, phosphorylase B, pyruvate kinase, creatinine kinase and phosphogluco-mutase. All the above proteins showed peptide bond cleavage as evidenced by the presence of lower molecular weight peptide fragments on the SDS gel.

Example 12

Determination of Site of Cleavage of Asparaginase by N,N-diethylaminophopylbis(3-hydroxypropyl) phosphine.

To confirm that the primary site of peptide bond cleavage is at cystine residues, purified asparaginase was used as a model protein. The intact polypeptide chain of asparaginase has a molecular weight of 34,000. Cysteine residues occur at residue 76 and 105 in the chain of 321 amino acids. Figure 3 is a representation of the asparaginase structure and the SDS gel after reaction of asparaginase with N-N-diethylaminopropylbis(3-hydroxypropyl)phosphine. The sizes of the peptide fragments were determined from a standard curve plot. The peptide fragments seen after reaction correspond to molecular weights of 26,500, 23,500, 19,000 and 8,000 daltons. In addition, a low molecular weight band (approximately 3000 daltons) is seen. The reaction does not go to completion and considerable parent polypeptide (34K) remains uncleaved.

If the phosphine cleaves all the possible cysteine sites simultaneously, the expected fragments from the primary sequence are 2971, 8055, 11,026, and 23,054 daltons. This obviously is not the case since we see a 26,500 fragment present in the gel pattern. Thus a partial cleavage of the polypeptide occurs as well.

Partial cleavage of the asparaginase with a hit on a single cysteine residue results in the following possible fragments, stating from the amino- terminal end: 8055, 26,025, 11,026, and 23,054. This still does not account for the lack of the 11,025 fragment and the presence of the 19,000 molecular weight band. Therefore, a possible explanation may be advanced as follows: the 11,025 if crosslinked with the 8055 fragment will give a 19,000 fragment. Assuming that the reaction occurs to completion there would remain the 8055 from the simultaneous hit of both cysteine residues but no remaining 11,025 since they are bound up by the excess 8055 fragment. This is consistent with the gel data since a 8055 fragment is visible in the gel.

It appears from the band intensity that the 23,054 fragment is present in approximately twice the concentration of the other fragements. This is likely if the assumption is valid that the two single and simultaneous hit occurs in equal proportions.

Example 13

Peptide Bond Cleavage Ability of Other Tertiary Organophosphines

The ability of tertiary organophospines with and without nucleophilic side-chains to cleave peptides was evaluated. Using the basic procedure for digestion outlined above, it was found that tertiary organophosphines with nucleophilic side chains cleave proteins containing cysteine residues while tertiary organophosphines without nucleophilic side chains act as reducing agents but do not cleave such proteins.

For example, tertiary organophosphines with tris-carboxy- ethyl, tris-n-butyl, tris-isobutyl or tri-cyanoethyl side chains, show no sign of cleaving peptide bonds. The results of such an experiment using BSA and tris-(isobutyl)phosphine and depicted in Figure 2. Only the parent, 66K, protein is seen after the reaction.

The nucleophilic tertiary organophosphines of the present invention selectively cleave proteins containing cysteine residues at the cysteine residue sites. Proteins containing numerous cysteine -cysteine sequences, such as bovine serum albumin, are all readily cleaved by the compounds of this invention. Other proteins with fewer cysteine residues may require application of heat to the digestion reaction to push the reaction to completion. The cleavage reaction of the compounds of this invention occurs over a wide pH range (4-12) and can be adjusted for optimal results by one skilled in the art to which this invention pertains. The reaction can be performed in various approporiate aqueous buffer solutions.

Due to the selectively of the cleavage by the compounds of this invention for cysteine residue sites, these compounds are very useful as a reagents for mapping of protein sequences and protein structural analysis. Such mapping and analysis can be performed using the digestion procedures described above.

Since keratins contain a high percentage of cysteine residues, treatment of keratins with the compounds of this invention will solubilize them. By way of example and not of limitation, feathers, callus tissue, hair and nails can be solubilized by these nucleophilic tertiary organophosphines. Such solubilization can be useful as a dehairing agent prior to tanning of animal hides and as an agent for the removal of ingrown toenails or callus tissue.

The compounds of this invention also may have antibacterial, antiviral and antifungal activity since these compounds cleave cystine containing proteins.

With the current advances in biotechnology including the engineering of specific proteins, the compounds of this invention will be useful as additional, selective tools in the modification of proteins by selective cleavage of protein.

The foregoing specified uses for the compounds of this invention are set forth as examples and are not intended, by their inclusion, to limit the scope of this invention in any way. Other uses for these compounds will be evident to those skilled in the art with the benefit of the disclosure contained herein and such uses shall therefore be within the scope of this invention.

**Claims**

**Claims for the following Contracting States : AT, BE, CH, DE, FR, GB, GR, IT, LI, NL, SE**

1. A method for disrupting peptide bonds at cysteine residues in polypeptide chains and proteins containing intact, inter- or intra-chain disulfide bonds which comprises treating with organic phosphines having nucleophilic side chains, said phosphines being selected from the group consisting of tertiary organophosphines of the general formula

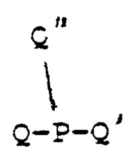

where Q, Q' and Q" are hydroxylalkyl, hydroxyalkoxyalkyl, poly(hydroxyalkyl), aminoalkyl or cycloaminoalkyl; where alkyl is $C_1$-$C_4$, the amine is primary , secondary or tertiary and the cycloamine is a 5 or 6 membered heterocyclic ring containing nitrogen.

2. A method according to claim 1 wherein said tertiary phosphines are selected from compounds of the formula

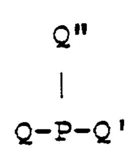

where Q is

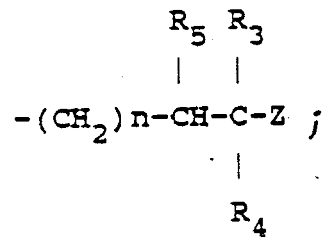

Q' is Q or Q'';
Q'' is

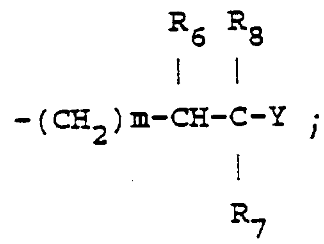

Y and Z are $-NR_{10}R_{11}$ or $-OR_{12}$;
$R_{10}$ and $R_{11}$ taken with the N form imidazolyl, pyrridonyl, or pyridyl rings;
$R_{12}$ is

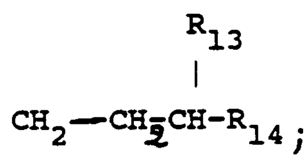

$R_{13}$ is H or OH;
$R_{14}$ is $-(CH_2)_t-CH_2-OH$;
n,m and t are independently 0-3;
$R_3$-$R_8$ are independently H or alkyl ($C_1$-$C_4$);
with the provisos that: Z and Y are not the same for any given compound.

3. A method according to claim 2 wherein the organic phosphine is 3-N,N-diethylaminopropylbis-3-imidazolylpropyl)phosphine.

4. Tertiary phosphines selected from compounds of the formula

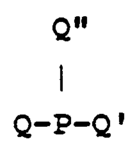

where Q is

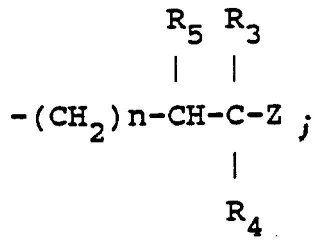

Q' is Q or Q'';
Q'' is

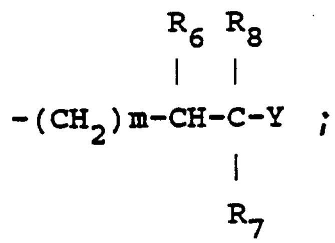

Y and Z are $-NR_{10}R_{11}$ or $-OR_{12}$;
$R_{10}$ and $R_{11}$ taken with the N form imidazolyl, pyrridonyl or pyridyl rings;
$R_{12}$ is

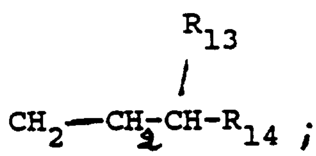

$R_{13}$ is H or OH;
$R_{14}$ is $-(CH_2)_t-CH_2-OH$;
n,m and t are independently 0-3;
$R_3$-$R_8$ are indepently H or alkyl ($C_1$-$C_4$);
with the provisos that: Z and Y are not the same for any given compound

**5.** The compound 3-N,N-diethylaminopropylbis (3-imidazolylpropyl)phosphine.

**6.** The compound 3-N,N-diethylaminopropylbis (2-hydroxy-ethyloxy-3-propyl)phosphine.

**7.** The compound 3-imidazolylpropylbis(3-hydroxy-propyl)phosphine.

**8.** The compound 3-N, N-diethylaminopropylbis (1,2-dihydroxypropyloxy-3-propyl)phosphine.

**9.** The compound N-pyrollidonylethylbis[1,2-dihydroxypropyloxy-3-propyl)phosphine.

**10.** The compound 3-hydroxypropylbis[3-imidazolypropyl)phosphine.

**Claims for the following Contracting State : ES**

**1.** A method for disrupting peptide bonds at cysteine residues in polypeptide chains and proteins containing intact, inter- or intra-chain disulfide bonds which comprises treating with organic phosphines having nucleophilic side chains, said phosphines being selected from the group consisting of tertiary organophosphines of the general formula

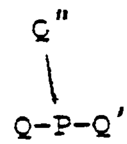

where Q, Q' and Q" are hydroxylalkyl, hydroxyalkoxyalkyl, poly(hydroxyalkyl), aminoalkyl or cycloaminoalkyl; where alkyl is C1-C4, the amine is primary , secondary or tertiary and the cycloamine is a 5 or 6 membered heterocyclic ring containing nitrogen.

**2.** A method according to claim 1 wherein said tertiary phosphines are selected from compounds of the formula

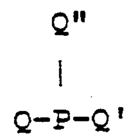

where Q is

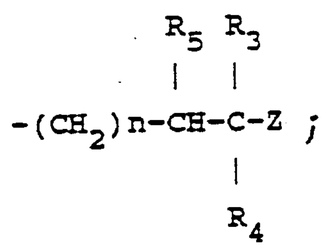

Q' is Q or Q";
Q" is

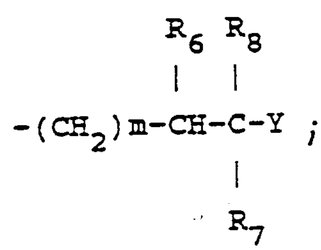

Y and Z are $-NR_{10}R_{11}$ or $-OR_{12}$;
$R_{10}$ and $R_{11}$ taken with the N form imidazolyl, pyrridonyl, or pyridyl rings;
$R_{12}$ is

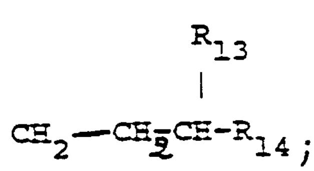

$R_{13}$ is H or OH;
$R_{14}$ is $-(CH_2)_t-CH_2-OH$;
n,m and t are independently 0-3;
$R_3$-$R_8$ are independently H or alkyl ($C_1$-$C_4$);
with the provisos that: Z and Y are not the same for any given compound.

3. A method according to claim 2 wherein the organic phosphine is 3-N,N-diethylaminopropylbis-3-imidazolylpropyl)phosphine.

4. A process for preparing tertiary phosphines selected from compounds of the formula

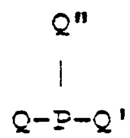

where Q is

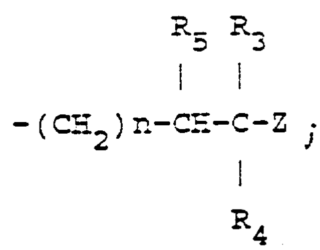

Q' is Q or Q'';
Q'' is

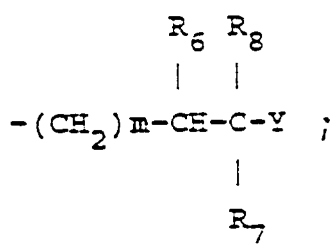

Y and Z are $-NR_{10}R_{11}$ or $-OR_{12}$;
$R_{10}$ and $R_{11}$ taken with the N form imidazolyl, pyrridonyl or pyridyl rings;
$R_{12}$ is

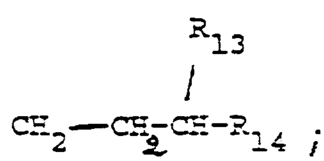

$R_{13}$ is H or OH;
$R_{14}$ is $-(CH_2)_t-CH_2-OH$;
n,m and t are independently 0-3;
$R_3$-$R_8$ are indepently H or alkyl ($C_1$-$C_4$);
with the provisos that: Z and Y are not the same for any given compound, said process being characterized in that phosphine ($PH_3$) is reacted in a pressurized vessel with a first terminally ethylenic unsaturated amine or alcohol in the presence of a free radical initiator to obtain a primary or secondary phosphine, which is separated from the reaction mixture by distillation, dissolved in a suitable solvent and reacted in an inert atmosphere with a second terminally ethylenically unsaturated alcohol or amine, again in the presence of a free radical initiator.

5. The process according to claim 4 which produces 3-N,N-diethylaminopropylbis(3-imidazolylpropyl)-phosphine.

6. The process according to claim 4 which produces 3-N,N-diethylaminopropylbis(2-hydroxy-ethyloxy-3-propyl)phosphine

7. The process according to claim 4 which produces 3-imidazolylpropylbis(3-hydroxy-propyl)phosphine.

8. The process according to claim 4 which produces 3-N,N-diethylaminopropylbis(1,2-dihydroxypropyloxy-3-propyl) phosphine.

9. The process according to claim 4 which produces N-pyrollidonylethylbis[1,2-dihydroxypropyloxy-3-propyl)phosphine.

10. The process according to claim 4 which produces 3-hydroxypropylbis[3-imidazolypropyl)phosphine.

**Patentansprüche**

**Patentansprüche für folgende Vertragsstaaten : AT, BE, CH, DE, FR, GB, GR, IT, LI, NL, SE**

1. Verfahren zum Aufbrechen von Peptidbindungen an Cysteinresten in Polypeptidketten und Proteinen, die intakte Inter- oder Intraketten-Disulfidbindungen enthalten, umfassend die Behandlung mit organischen Phosphinen mit nukleophilen Seitenketten, wobei die Phosphine ausgewählt sind aus der Gruppe, die aus tertiären Organophosphinen der allgemeinen Formel

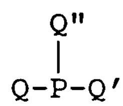

besteht, worin Q, Q' und Q" Hydroxyalkyl, Hydroxyalkoxyalkyl, Poly(hydroxyalkyl), Aminoalkyl oder Cycloaminoalkyl sind; worin Alkyl C1-C4 ist, das Amin primär, sekundär oder tertiär ist und das Cycloamin ein 5- oder 6-gliedriger heterocyclischer Ring, der Stickstoff enthält, ist.

2. Verfahren nach Anspruch 1, worin die tertiären Phosphine ausgewählt sind aus Verbindungen der Formel

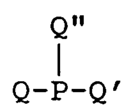

worin Q

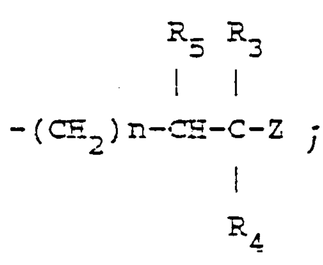

ist;
Q' Q oder Q" ist;
Q"

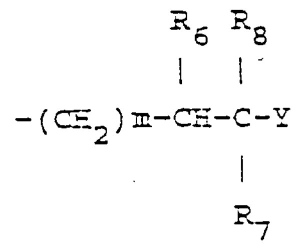

ist;
Y und Z $-NR_{10}R_{11}$ oder $-OR_{12}$ sind;
$R_{10}$ und $R_{11}$ mit dem N genommen Imidazolyl-, Pyrridonyl- oder Pyridylringe bilden;
$R_{12}$

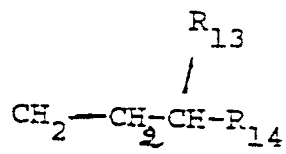

ist;
$R_{13}$ H oder OH ist;
$R_{14}$ $-(CH_2)_t-CH_2-OH$ ist;
n, m und t unabhängig 0-3 sind;
$R_3$-$R_8$ unabhängig H oder $(C_1-C_4)$-Alkyl sind;
mit der Maßgabe, daß Z und Y für irgendeine gegebene Verbindung nicht gleich sind.

3. Verfahren nach Anspruch 2, worin das organische Phosphin 3-N,N-Diethylaminopropylbis(3-imidazolylpropyl)phosphin ist.

4. Tertiäres Phosphin, ausgewählt aus Verbindungen der Formel

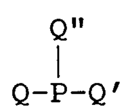

worin Q

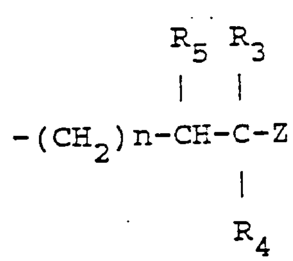

ist;
Q' Q oder Q'' ist;
Q''

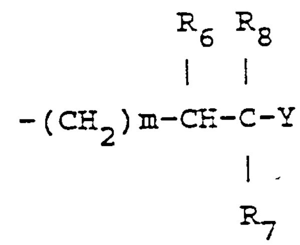

ist;
Y und Z $-NR_{10}R_{11}$ oder $-OR_{12}$ sind;
$R_{10}$ und $R_{11}$ mit dem N genommen Imidazolyl-, Pyrridonyl- oder Pyridylringe bilden;
$R_{12}$

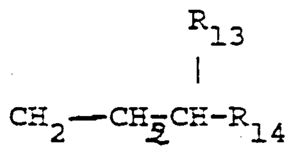

ist;
$R_{13}$ H oder OH ist;
$R_{14}$ $-(CH_2)_t-CH_2-OH$ ist;
n, m und t unabhängig 0-3 sind;
$R_3$-$R_8$ unabhängig H oder $(C_1$-$C_4)$-Alkyl sind;
mit der Maßgabe, daß Z und Y für irgendeine gegebene Verbindung nicht gleich sind.

**5.** Die Verbindung 3-N,N-Diethylaminopropylbis(3-imidazolylpropyl)phosphin.

**6.** Die Verbindung 3-N,N-Diethylaminopropylbis(2-hydroxyethyloxy-3-propyl)phosphin.

**7.** Die Verbindung 3-Imidazolylpropylbis(3-hydroxypropyl)phosphin.

**8.** Die Verbindung 3-N,N-Diethylaminopropylbis(1,2-dihydroxypropyloxy-3-propyl)phosphin.

**9.** Die Verbindung N-Pyrollidonylethylbis(1,2-dihydroxypropyloxy-3-propyl)phosphin.

**10.** Die Verbindung 3-Hydroxypropylbis(3-imidazolylpropyl)phosphin.

**Patentansprüche für folgenden Vertragsstaat : ES**

**1.** Verfahren zum Aufbrechen von Peptidbindungen an Cysteinresten in Polypeptidketten und Proteinen, die intakte Inter- oder Intraketten-Disulfidbindungen enthalten, umfassend die Behandlung mit organischen Phosphinen mit nukleophilen Seitenketten, wobei die Phosphine ausgewählt sind aus der Gruppe, die aus tertiären Organophosphinen der allgemeinen Formel

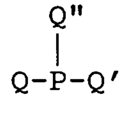

besteht, worin Q, Q' und Q" Hydroxyalkyl, Hydroxyalkoxyalkyl, Poly(hydroxyalkyl), Aminoalkyl oder Cycloaminoalkyl sind; worin Alkyl C1-C4 ist, das Amin primär, sekundär oder tertiär ist und das Cycloamin ein 5- oder 6-gliedriger heterocyclischer Ring, der Stickstoff enthält, ist.

2. Verfahren nach Anspruch 1, worin die tertiären Phosphine ausgewählt sind aus Verbindungen der Formel

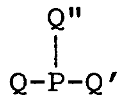

worin Q

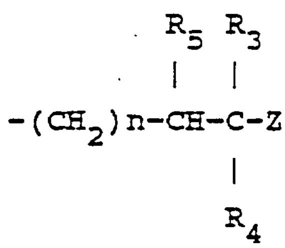

ist;
Q' Q oder Q" ist;
Q"

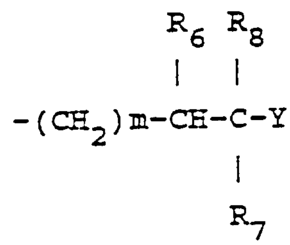

ist;
Y und Z $-NR_{10}R_{11}$ oder $-OR_{12}$ sind;
$R_{10}$ und $R_{11}$ mit dem N genommen Imidazolyl-, Pyrridonyl- oder Pyridylringe bilden;
$R_{12}$

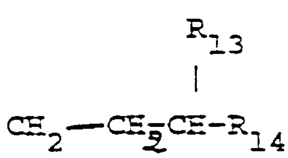

ist;
$R_{13}$ H oder OH ist;
$R_{14}$ $-(CH_2)_t-CH_2-OH$ ist;
n, m und t unabhängig 0-3 sind;
$R_3$-$R_8$ unabhängig H oder ($C_1$-$C_4$)-Alkyl sind;
mit der Maßgabe, daß Z und Y für irgendeine gegebene Verbindung nicht gleich sind.

3. Verfahren nach Anspruch 2, worin das organische Phosphin 3-N,N-Diethylaminopropylbis(3-imidazolylpropyl)phosphin ist.

4. Verfahren zur Herstellung von tertiären Phosphinen, die ausgewählt sind aus Verbindungen der Formel

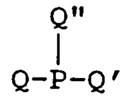

worin Q

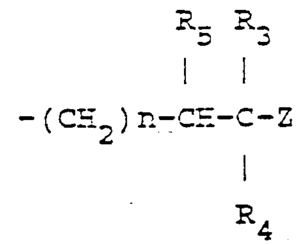

ist;
Q' Q oder Q'' ist;
Q''

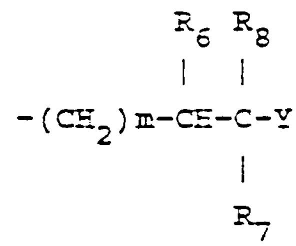

ist;
Y und Z $-NR_{10}R_{11}$ oder $-OR_{12}$ sind;
$R_{10}$ und $R_{11}$ mit dem N genommen Imidazolyl-, Pyrridonyl- oder Pyridylringe bilden;
$R_{12}$

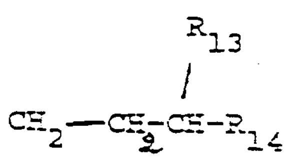

ist;
$R_{13}$ H oder OH ist;
$R_{14}$ $-(CH_2)_t-CH_2-OH$ ist;
n, m und t unabhängig 0-3 sind;
$R_3$-$R_8$ unabhängig H oder ($C_1$-$C_4$)-Alkyl sind;
mit der Maßgabe, daß Z und Y für irgendeine gegebene Verbindung nicht gleich sind,
wobei das Verfahren dadurch gekennzeichnet ist, daß Phosphin ($PH_3$) in einem unter Druck gesetzten Gefäß mit einem ersten terminal ethylenisch ungesättigten Amin oder Alkohol in Anwesenheit eines freie Radikale bildenden Initiators umgesetzt wird, um ein primäres oder sekundäres Phosphin zu erhalten, das durch Destillation aus der Reaktionsmischung abgetrennt wird, in einem geeigneten Lösungsmittel gelöst wird und in einer Inertatmosphäre wieder in Gegenwart eines freie Radikale bildenden Initiators mit einem zweiten terminal ethylenisch ungesättigten Alkohol oder Amin umgesetzt wird.

**5.** Verfahren nach Anspruch 4, das 3-N,N-Diethylaminopropylbis(3-imidazolylpropyl)phosphin erzeugt.

**6.** Verfahren nach Anspruch 4, das 3-N,N-Diethylaminopropylbis(2-hydroxy-ethyloxy-3-propyl)phosphin erzeugt.

**7.** Verfahren nach Anspruch 4, das 3-Imidazolylpropylbis(3-hydroxypropyl)phosphin erzeugt.

**8.** Verfahren nach Anspruch 4, das 3-N,N-Diethylaminopropylbis(1,2-dihydroxypropyloxy-3-propyl)-phosphin erzeugt.

**9.** Verfahren nach Anspruch 4, das N-Pyrollidonylethylbis(1,2-dihydroxypropyloxy-3-propyl)phosphin erzeugt.

**10.** Verfahren nach Anspruch 4, das 3-Hydroxypropylbis(3-imidazolylpropyl)phosphin erzeugt.

**Revendications**

**Revendications pour les Etats contractants suivants : AT, BE, CH, DE, FR, GB, GR, IT, LI, NL, SE**

**1.** Procédé pour rompre les liaisons peptidiques au niveau des résidus cystéine dans les chaînes polypeptidiques et les protéines contenant des liaisons disulfure intactes, inter ou intra-chaîne, qui comprend le traitement par des phosphines organiques ayant des chaînes latérales nucléophiles, lesdites phosphines étant choisies dans le groupe constitué d'organophosphines tertiaires de formule générale

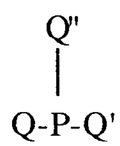

dans laquelle Q, Q' et Q'' sont hydroxyalkyle, hydroxyalcoxyalkyle, poly(hydroxyalkyle), aminoalkyle ou cycloaminoalkyle ; dans lesquels l'alkyle est en $C_1$-$C_4$, l'amine est primaire, secondaire ou tertiaire et la cycloamine est un groupe hétérocyclique à 5 ou 6 chaînons contenant de l'azote.

**2.** Procédé selon la revendication 1, dans lequel les phosphines tertiaires sont choisies parmi les composés de formule

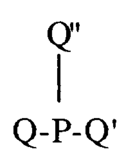

dans laquelle Q est

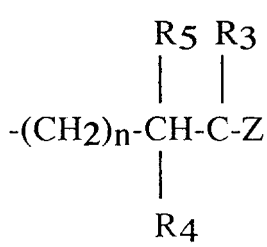 ;

Q' est Q ou Q'';

Q'' est

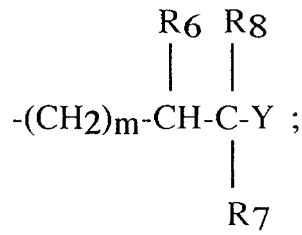

Y et Z sont $-NR_{10}R_{11}$ ou $-OR_{12}$ ;
$R_{10}$ et $R_{11}$ pris avec N forment un cycle imidazolyle, pyrridonyle ou pyridyle ;
$R_{12}$ est

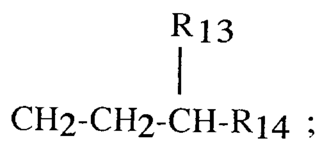

$R_{13}$ est H ou OH ;
$R_{14}$ est $-(CH_2)_t-CH_2-OH$ ;
n, m et t sont indépendamment 0-3 ;
$R_3$ à $R_8$ sont indépendamment H ou alkyle ($C_1$-$C_4$) ;
avec la condition selon laquelle : Z et Y ne sont pas identiques pour un composé donné quelqu'il soit.

3. Procédé selon la revendication 2 dans lequel la phosphine organique est la 3-N,N-diéthylaminopropyl-bis-3-imidazolylpropyl)phosphine.

4. Phosphines tertiaires choisies parmi les composés de formule

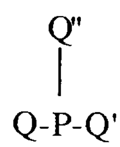

dans laquelle Q est

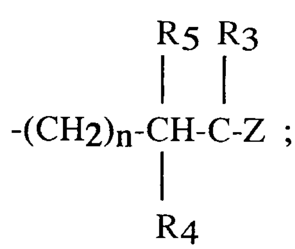

Q' est Q ou Q'' ;
Q'' est

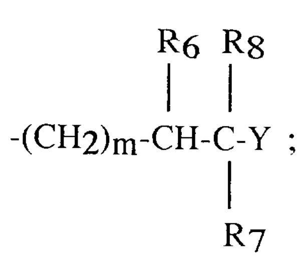

Y et Z sont $-NR_{10}R_{11}$ ou $-OR_{12}$ ;
$R_{10}$ et $R_{11}$ forment N un cycle imidazolyle, pyrridonyle ou pyridyle ;
$R_{12}$ est

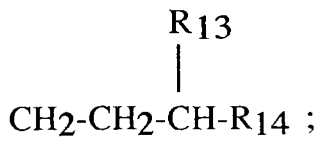

$R_{13}$ est H ou OH ;
$R_{14}$ est $-(CH_2)_t-CH_2-OH$ ;
n, m et t sont indépendamment 0-3 ;
$R_3$ à $R_8$ sont indépendamment H ou alkyle ($C_1$-$C_4$) ;
avec la condition selon laquelle : Z et Y ne sont pas identiques pour un composé donné quelqu'il soit.

**5.** Composé 3-N,N-diéthylaminopropylbis(3-imidazolylpropyl)phosphine.

**6.** Composé 3-N,N-diéthylaminopropylbis(2-hydroxyéthyloxy-3-propyl)phosphine.

**7.** Composé 3-imidazolylpropylbis(3-hydroxypropyl)phosphine.

**8.** Composé 3-N,N-diéthylaminopropylbis(1,2-dihydroxypropyloxy-3-propyl)phosphine.

**9.** Composé N-pyrollidonyléthylbis[1,2-dihydroxypropyloxy-3-propyl)phosphine.

**10.** Composé 3-hydroxypropylbis[3-imidazolypropyl)phosphine.

**Revendications pour l'Etat contractant suivant : ES**

**1.** Procédé pour rompre les liaisons peptidiques au niveau des résidus cystéine dans les chaînes polypeptidiques et les protéines contenant des liaisons disulfure intactes, inter ou intra-chaîne, qui comprend le traitement par des phosphines organiques ayant des chaînes latérales nucléophiles, lesdites phosphines étant choisies dans le groupe constitué d'organophosphines tertiaires de formule générale

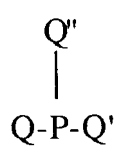

dans laquelle Q, Q' et Q'' sont hydroxyalkyle, hydroxyalcoxyalkyle, poly(hydroxyalkyle), aminoalkyle ou cycloaminoalkyle ; dans lesquels l'alkyle est en $C_1$-$C_4$, l'amine est primaire, secondaire ou tertiaire et la cycloamine est un groupe hétérocyclique à 5 ou 6 chaînons contenant de l'azote.

**2.** Procédé selon la revendication 1, dans lequel les phosphines tertiaires sont choisies parmi les composés de formule

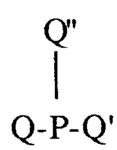

dans laquelle Q est

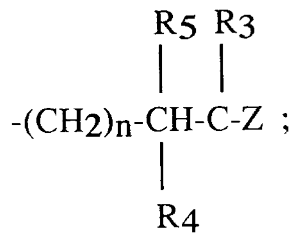

Q' est Q ou Q'' ;
Q'' est

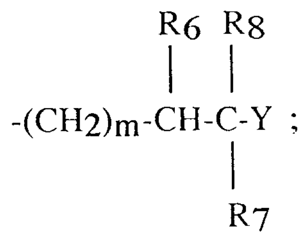

Y et Z sont $-NR_{10}R_{11}$ ou $-OR_{12}$ ;
$R_{10}$ et $R_{11}$ forment avec N un cycle imidazolyle, pyrridonyle ou pyridyle ;
$R_{12}$ est

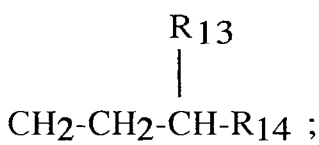

$R_{13}$ est H ou OH ;
$R_{14}$ est $-(CH_2)_t-CH_2-OH$ ;
n, m et t sont indépendamment 0-3 ;
$R_3$ à $R_8$ sont indépendamment H ou alkyle ($C_1$-$C_4$) ;
avec la condition selon laquelle : Z et Y ne sont pas identiques pour un composé donné quelqu'il soit.

**3.** Procédé selon la revendication 2 dans lequel la phosphine organique est la 3-N,N-diéthylaminopropyl-bis-3-imidazolylpropyl)phosphine.

**4.** Procédé pour préparer des phosphines tertiaires choisies parmi les composés de formule

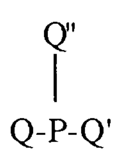

dans laquelle Q est

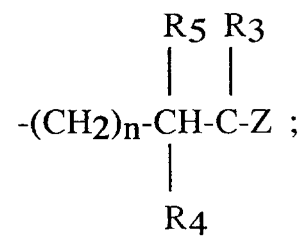

Q' est Q ou Q'';
Q'' est

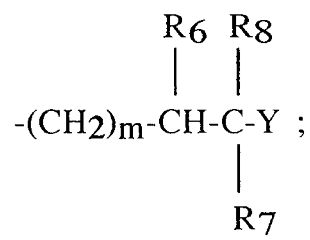

Y et Z sont $-NR_{10}R_{11}$ ou $-OR_{12}$ ;
$R_{10}$ et $R_{11}$ forment avec N un cycle imidazolyle, pyrridonyle ou pyridyle ;
$R_{12}$ est

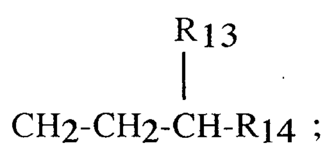

$R_{13}$ est H ou OH ;
$R_{14}$ est $-(CH_2)_t-CH_2-OH$ ;
n, m et t sont indépendamment 0-3 ;
avec la condition selon laquelle : Z et Y ne sont pas identiques pour un composé donné quelqu'il soit, ledit procédé étant caractérisé en ce que la phosphine ($PH_3$) est mise à réagir dans un récipient pressurisé avec une première amine ou un premier alcool, insaturé(e) éthylèniquement à son extrémité, en présence d'un initiateur de radicaux libres pour obtenir une phosphine primaire ou secondaire, qui est séparée du mélange réactionnel par distillation, dissoute dans un solvant approprié et mise à réagir dans une atmosphère inerte avec un second alcool ou une seconde amine, insaturé(e) éthylèniquement à son extrémité, à nouveau en présence d'un initiateur de radicaux libres.

5. Procédé de la revendication 4 qui produit la 3-N,N-diéthylaminopropylbis(3-imidazolylpropyl)phosphine.

6. Procédé de la revendication 4 qui produit la 3-N,N-diéthylaminopropyibis(2-hydroxyéthyloxy-3-propyl)-phosphine.

7. Procédé de la revendication 4 qui produit la 3-imidazolylpropylbis(3-hydroxypropyl)phosphine.

8. Procédé de la revendication 4 qui produit la 3-N,N-diéthylaminopropylbis(1,2-dihydroxypropyloxy-3-propyl)phosphine.

9. Procédé de la revendication 4 qui produit la N-pyrollidonyléthylbis[1,2-dihydroxypropyloxy-3-propyl)-phosphine.

10. Procédé de la revendication 4 qui produit la 3-hydroxypropylbis[3-imidazolypropyl)phosphine.

# MECHANISM OF ACTION STUDIES USING HUMAN HAIR

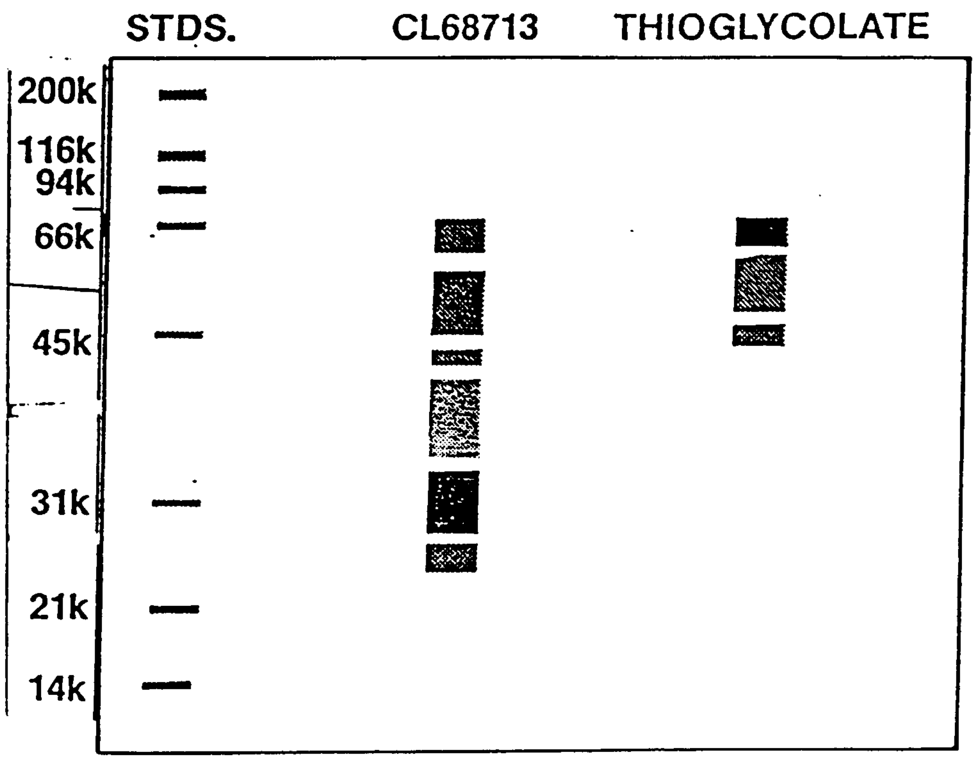


Protein fragments produced by digestion of hair with phosphine are smaller than those seen with thioglycolate, implying a different or additional mechanism of action

Fig. 1

# ELECTROPHORETIC STUDY BOVINE SERUM ALBUMIN M.WT = 66 K

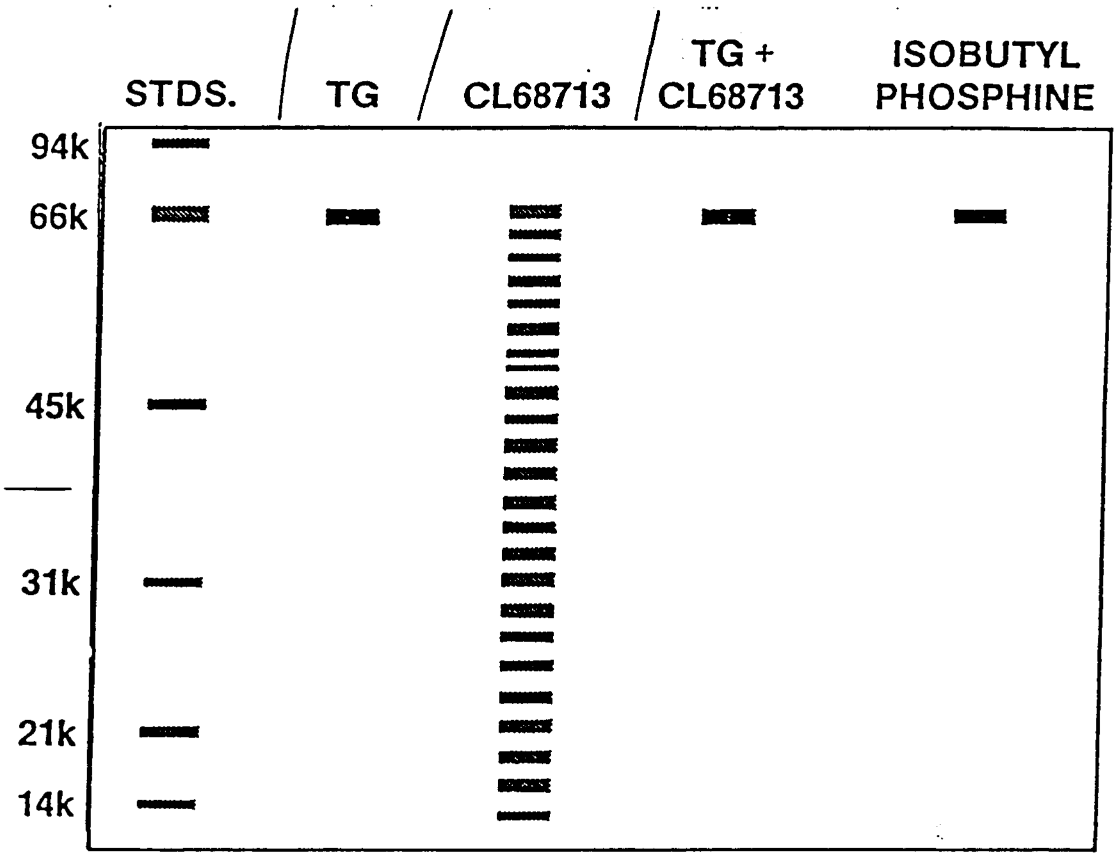


Fig. 2

# ELEOTROPHORETIC STUDY

Asparaginase: A protein containing 2 cysteines
M.W. = 34k

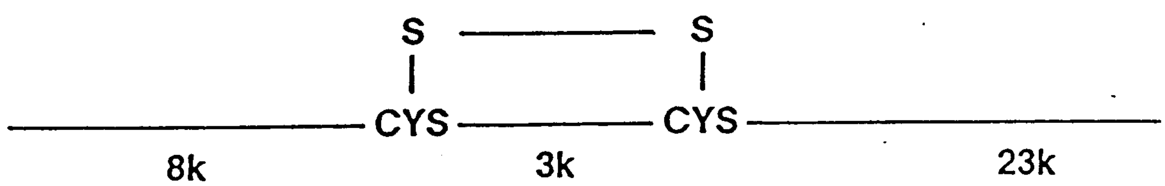


Phosphine cleavage could occur at one or both cysteines

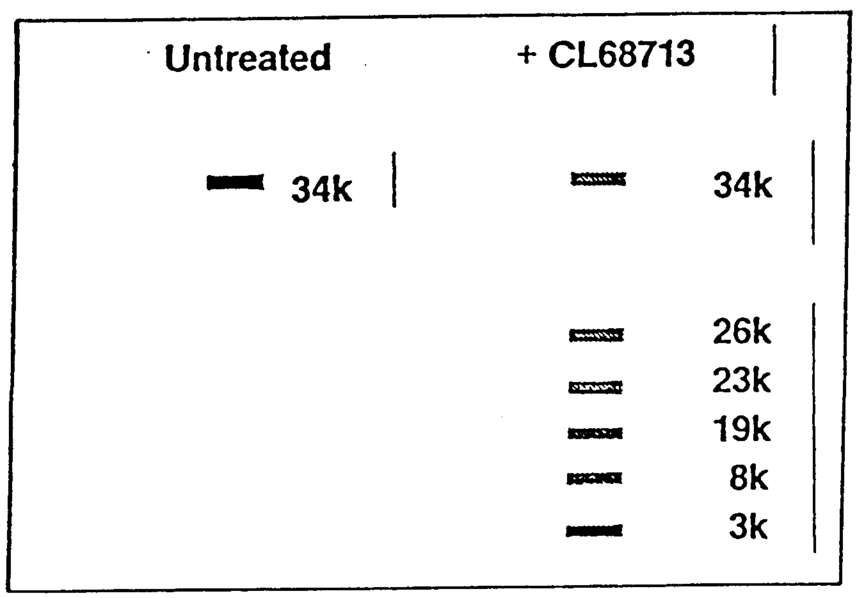


— All fragments accounted for except 11k
— 19k fragment may result from cross-linking of 8k + 11k fragments

Fig.3